# EUROPÄISCHE PATENTSCHRIFT

(11) **EP 3 517 600 B1**
(45) Veröffentlichungstag und Bekanntmachung des Hinweises auf die Patenterteilung: **24.02.2021**
(21) Anmeldenummer: 18000410.3
(22) Anmeldetag: 30.04.2018
(51) Int. Cl.: C12M 1/00

(54) **MÄANDER-BIOREAKTOR UND VERFAHREN FÜR DIE ISOLIERUNG UND VERMEHRUNG VON ZELLEN AUS GEWEBETEILEN VON TUMOREN, METASTASEN UND ANDEREN GEWEBEN**
MEANDER BIOREACTOR AND METHOD FOR THE ISOLATION AND PROLIFERATION OF CELLS FROM PARTS FROM TUMOURS, METASTASES AND OTHER TISSUES
BIORÉACTEUR MÄANDER ET PROCÉDÉ D'ISOLATION ET DE REPRODUCTION DE CELLULES PROVENANT DES PARTIES TISSUS DES TUMEURS, DES MÉTASTASES ET D'AUTRES TISSUS

(30) Priorität: 24.01.2018 DE 102018000561
(43) Veröffentlichungstag der Anmeldung: 31.07.2019
(73) Patentinhaber: Zellwerk GmbH, 16727 Oberkrämer OT Eichstädt (DE)
(72) Erfinder: Hoffmeister, Hans, 32549 Bad Oeynhausen (DE)

(56) Entgegenhaltungen:
- EP-A1- 2 543 719
- US-A1- 2004 058 437
- KLAPPER J A ET AL: "Single-pass, closed-system rapid expansion of lymphocyte cultures for adoptive cell therapy", JOURNAL OF IMMUNOLOGICAL METHODS, ELSEVIER SCIENCE PUBLISHERS B.V.,AMSTERDAM, NL, Bd. 345, Nr. 1-2, 30. Juni 2009 (2009-06-30), Seiten 90-99, XP026132879, ISSN: 0022-1759, DOI: 10.1016/J.JIM.2009.04.009 [gefunden am 2009-04-21]

## Beschreibung

Mäander-Bioreaktor und Verfahren für die Isolierung und Vermehrung von Zellen aus Gewebeteilen von Tumoren, Metastasen und anderen Geweben Die Erfindung betrifft einen neuartigen Bioreaktor-Typ, mit dem es gelingt, in einem einzigen geschlossenen Verfahrensschritt aus kleingeschnittenen Gewebestücken bestimmte Zellen sowohl in das im Bioreaktor befindliche Medium auswachsen zu lassen, als auch die Zellen gleichzeitig zu aktivieren, zu vermehren und dann von Geweberesten abzutrennen. Insbesondere können auf diese Weise tumorinfiltrierende autologe T- Lymphozyten (TIL) aus Tumorgewebe, Metastasengewebe, deren Umgebungsgewebe und aus Lymphknoten, die von Tumorzellen befallenen sind, gewonnen werden.

Die DE 695 34 132 T2 beschreibt eine Zellkulturvorrichtung, die einen Behälter mit einem Zellkulturabteil umfasst, wobei der Behälter für die Zellvermehrung von einer unteren, gasdurchlässigen, bevorzugt für Sauerstoff durchlässigen Schicht und einer oberen Folie, die selektiv für Substanzen einer vorgegebenen Größe durchlässig und für Zellen undurchlässig sind, begrenzt wird. Der Behälter ist dafür ausgelegt, ein Kulturmedium zwischen der oberen Folie und der unteren gasdurchlässigen Schicht aufzunehmen. Der Behälter besteht aus
- einem Basalmediumabteil , von dem sich mindestens ein Teil über der oberen Folie befindet, wobei ein Basalmedium auf der oberen Folie liegen kann;
- nur einer einzigen Zugangsöffnung zu dem Zellkulturabteil ;
- einer Zugangsöffnung zu dem Basalmediumabteil;
   und
- einem gasdurchlässigen Schichtträger, der unterhalb der gasdurchlässigen Schicht angeordnet ist und mit dieser teilweise in Kontakt steht, wodurch der größte Teil der gasdurchlässigen Schicht in einer im Wesentlichen horizontalen Lage gehalten wird, so dass sich Zellen einer Suspension entlang des horizontalen Abschnitts der gasdurchlässigen Schicht verteilen können und ein Gasaustausch mit dem Zellkulturabteil nicht wesentlich behindert wird. Die Versorgung der Zellen mit Medium erfolgt ungeregelt, die Überströmung der Zellen ist nicht gerichtet.

In der DE 693 32 374 T2 wird ein Bioreaktor zum Kultivieren von Humanzellen und/oder -gewebe, einschließlich humaner Stammzellen oder humaner hämatopoetischer Zellen beschrieben, der aus einem Gehäuse , das eine Zellkulturkammer definiert, in die Humanzellen und/oder gewebe, einschließlich humaner Stammzellen oder humaner hämatopoetischer Zellen, eingebracht und worin sie kultiviert werden können, wobei diese Zellkulturkammer so ausgebildet ist, das sie eine Vielzahl von Einlassöffnungen im Wesentlichen äquidistant zu einer Vielzahl von Auslassöffnungen enthält, damit Zellkulturmedium hindurchströmen kann, besteht. Der Bioreaktor beschreibt weiterhin Mittel zum Perfundieren eines flüssigen Zellkulturmediums durch die Zellkulturkammer; Mittel zum In-Kontakt-Bringen des flüssigen Zellkulturmediums, das durch die Zellkulturkammer perfundiert wird, mit einer Sauerstoffquelle, so dass das flüssige Zellkulturmedium, das durch die Zellkulturkammer perfundiert wird, mit Sauerstoff angereichert wird, wobei diese Mittel zum In-Kontakt-Bringen eine für Gas permeable und für Flüssigkeit impermeable Membran enthalten; und Mittel zum kontinuierlichen, periodischen oder diskontinuierlichen Ernten nicht adhärenter Zellen aus der Zellkulturkammer durch die Strömung des Kulturmediums.Die für das Gas permeable und für Flüssigkeit impermeable Membran wird so in das Gehäuse eingebaut, dass das Gehäuse in die Zellkulturkammer auf der einen Seite der Membran und in eine Gaskammer auf der anderen Seite der Membran unterteilt wird. Der Bioreaktor umfasst Mittel zum Perfundieren von Atmungsgasen durch diese Zellkultur, Mittel zum Zuführen der Atmungsgase zu der Gaskammer, wodurch die Atmungsgase durch die Membran strömen und dabei durch die Zellkulturkammer hindurchtreten und Mittel zum Abziehen verbrauchter Atmungsgase aus der Gaskammer.

Die Erfindung gemäß der DE 69614535 T2 betrifft allgemein eine Vorrichtung zum ex vivo Aufbewahren und zur ex vivo Züchtung von biologischen Zellen und insbesondere eine Vorrichtung dieser Art, die die Zellen in einer tragbaren Kassette aufbewahrt und züchtet, während ein steriles System aufrechterhalten wird, das von der äußeren Umwelt abgeschlossen ist.Die Vorrichtung zur ex vivo Aufbewahrung und Züchtung von biologischen Zellen, umfasst eine tragbare Zellwachstumskassette, die einschließt ein Gehäuse, eine Zellwachstumskammer innerhalb des Gehäuses, konfiguriert um eine Menge von biologischen Zellen und ein Wachstumsmedium zu tragen, wobei die Zellwachstumskammer eine Medium-Einlassöffnung und eine Medium-Auslassöffnung aufweist, einen Mediumbehälter, der mit der Medium-Einlassöffnung der Zellwachstumskammer verbunden und konfiguriert ist, ein Wachstumsmedium zu enthalten, einen Abfallbehälter, der mit der Medium-Auslassöffnung der Zellwachstumskammer verbunden und konfiguriert ist, von der Wachstumskammer abgelassenes Medium aufzunehmen, wobei die Zellwachstumskammer, der Mediumbehälter und der Abfallbehälter ein von der äußeren Umwelt abgeschlossenes steriles System bilden. Die Zellwachstumskammer enthält eine bevorzugt für Sauerstoff permeable und Flüssigkeiten impermeable Membran, die einen Teil der Zellwachstumskammer definiert und eine Gaskammer, die auf der der Zellwachstumskammer gegenüberliegenden Seite der Membran lokalisiert ist. Die tragbare Zellwachstumskassette schließt weiterhin einen Gaszufuhranschluss ein, der von einer Rückwand des Gehäuses getragen wird, und eine Gaszufuhrleitung, welche den Gaszufuhranschluss mit der Gaskammer verbindet, um so Gas zur Gaskammer zur Permeation durch die Membran zu der Zellwachstumskammer zu liefern.

Die Erfindung gemäß der CN 1699558 (A) betrifft eine großtechnische Kultivierungstechnologie für Killerzellen zur Induktion von Zellfaktoren für hämatopoetische Stammzellen unter Verwendung eines Bioreaktors, der das Erhalten von Nabelschnurblut-hämatopoetischen Stammzellen, das Induzieren der hämatopoetischen Stammzellen in DC-Zellen mit Cytokin, das Vervollständigen der Funktion des Tumorantigens zu präsentieren, die ausgewählte Aktivierung von Killer-T-Lymphozyten mit spezifischer Abtötungswirkung an Tumoren zu vervollständigen, umfasst. Die Herstellung des Gelose-Mikroträgers für den monoklonalen Antikörper (CD3McAb), die Aktivierung der CIK-Zellen und die kontinuierliche Kultivierung von CIK-Zellen im Bioreaktor werden verknüpft.

Die DE 10 2011 106 914 B4 / EP25 43 719 A1 offenbart einen Mäander-Bioreaktor zur dynamischen und stressfreien Expansion, Differenzierung und Ernte der Gesamtfraktion hämatopoetischer Zellen, Stammzellen, Progenitorzellen, aber auch von isolierten, stimulierten, aktivierten Fraktionen dieser Zellen, außer embryonalen Zellen, wobei der Bioreaktor im Perfusionsbetrieb arbeitet. Der Bioreaktor besteht aus einem Bioreaktorgefäß, dessen Bodenfläche mit versetzt angeordneten Trennwänden versehen ist, die eine mäanderartige Führung der Medien mit einer laminaren Strömung bewirken. Der Mäander-Bioreaktor kann über Absperrventile mit anderen Mäander-Bioreaktoren modulartig in Reihe geschaltet werden. Die Zellen bilden nach dem Zuführen in das Bioreaktorgefäß eine zusammenhängende, die Bodenfläche des Mäander-Bioreaktors nahezu bedeckende Zellschicht, wobei die Zellen untereinander vielfach engen Kontakt zueinander haben. Die bei der Expansion entstehenden Zellklumpen werden durch Rütteln aufgelöst und geerntet oder werden einem nachfolgenden modulartigen Bioreaktor mit einer der Expansionsrate des vorherigen Bioreaktors entsprechenden größeren Bodenfläche des Bioreaktorgefäßes zur weiteren Expansion zugeführt.

Eine Technologie, die mehrere Verfahrensschritte zur Isolierung und Vermehrung von Zellen aus Gewebeteilen von Tumoren, Metastasen und anderen Geweben integriert, ist aus dem Stand der Technik nicht bekannt. Es ist auch kein Bioreaktor beschrieben, mit dem Zellen und insbesondere TIL steriltechnisch sicher und aufgrund ihrer hohen Zytotoxizität in therapeutisch sinnvollen Mengen hergestellt werden können in keinem, im Stand der Technik dargestellten Bioreaktoren zur Züchtung von Gewebe oder Zellen wurden bisher ex- vivo aus Geweben Zellen gleichzeitig isoliert, gezüchtet, aktiviert, expandiert und geerntet.
Auch ist kein Bioreaktor zur Durchführung dieses Verfahrens aus dem Stand der Technik bekannt.
Aufgabe der Erfindung ist, ein Mäander- Bioreaktor und ein Verfahren zu schaffen, die es ermöglichen, dass aus kleingeschnittenen Gewebestücken (die aus anfallenden OP-Gewebeteilen oder aus Biopsien stammen) Zellen in einem geschlossenen Perfusions-Verfahren anfangs auswachsen, sich fortlaufend vermehren, dabei aktiviert werden und nach Erreichen einer bestimmten Zelldichte geerntet werden können. Insbesondere können nach dem Verfahren tumorinfiltrierende autologe T- Lymphozyten, sogenannte TIL, aber auch weitere Immunzellen z. B. tumorinfiltrierte NK-Zellen (TINK) hergestellt werden

Die Aufgabe der Erfindung wird durch ein Mäander- Bioreaktor und ein zweistufiges Verfahren für die ex- vivo Gewinnung von Zellen gelöst, die in der ersten Stufe des Verfahrens aus zerkleinerten Gewebeteilen in das im Bioreaktorgefäß (1) befindliche Medium auswachsen, gleichzeitig aktiviert und vermehrt werden und nach Erreichen einer bestimmten Dichte an ausgewachsenen und expandierten Zellen von den Gewebeteilen separiert werden. Die Ausgangs-Gewebe, die von Tumorzellen befallenen sind, stammen bevorzugt aus Anlass von Organtumor- oder Metastasen-Operationen oder Entfernung von deren unmittelbar umgebenden Geweben oder auch aus Lymphknoten. Das Verfahren in der ersten Stufe erfolgt in einem geschlossenen Mäander

Bioreaktorgefäß (1), das mit einem üblichen Medium gerichtet perfundiert und dessen Medium mit einem Gemisch aus AB Humanserum, Cytokinen, Antikörpern und irradiierten humanen Feederzellen zeitweilig supplementiert wird. Die kleingeschnittenen Gewebestücke eignen sich in besonderer Weise für die Gewinnung von tumorinfiltrierten T-Lymphozyten (TIL), aber auch für tumortinfiltrierte natural Killer- Zellen (TINK) Nach 7 bis 14 Tagen Expansionsdauer werden die ausgewachsenen und vermehrten TIL über den mit Siebgewebe (16) versehenen Auslassstutzen (15) des Bioreaktorgefäßes (1) von den Geweberesten abfiltriert und in reiner Form geerntet. Die so separierten TIL werden zentrifugiert und in Einfriermedium in Vials resuspendiert, aliquotiert und kryokonserviert. Mit entsprechend supplementierten Medien können auch tumorinfiltrierte NK-Zellen (TINK) und weitere Zellarten in dem Mäander- Bioreaktor kultiviert werden.

Für klinische Anwendungen werden in der zweiten Stufe kryokonservierte Vials zeitlich so aufgetaut, dass in einer zweiten Expansionsphase therapeutisch sinnvolle Mengen an TIL termingerecht produziert werden. Dazu werden nach Auswaschen vom Einfriermedium die TIL erneut in geeignetem Medium suspendiert. Die TIL-Suspension (50 bis 100 Mio. vitale Zellen) wird in einen weiteren betriebsbereit installierten Mäander-Bioreaktor transferiert. Diese zweite Prozessstufe zur weiteren Expansion von TIL wird in einem Bioreaktor durchgeführt, der dem beschriebenen Mäander-Bioreaktor der ersten Stufe gleicht, der aber eine größere Besiedlungsfläche im Verhältnis von 3 zu 1 bis 20 zu 1 besitzt. Für die weitere Vermehrung wird frisch supplementiertes Medium verwendet.Nach 12 bis 20 Tagen Expansion wachsen im Mäander- Bioreaktor des zweiten Schrittes regelmäßig mehr als 500 Mio. TIL hoch Die TIL werden geerntet, gewaschen und in NaCl- Lösung mit Human-Albumin resuspendiert. Die TIL-Suspension wird in einen Infusionsbeutel abgefüllt. Viablilität und Zellzahl werden am Tag der Zellernte nach Vorgabe der Pharmakopöe Sterilität, Markerprofil und parakrine Produktion der Zellen analysiert. Für 500 Mio. TIL werden max.100 ml NaCl Lösung mit Human-Albumin eingesetzt. Die TIL-Suspension wird bei Raumtemperatur gehalten und sofort zum klinischen Partner transportiert. Die Applikation der TIL muss spätestens nach 12 bis 24 Stunden erfolgen. Überschüssige Mengen an TIL werden kryokonserviert. Sie stehen für später notwendige werdende Herstellung von weiteren Dosen an TIL zur Verfügung

In einer Auslegung des erfinderischen Verfahrens können die vermehrten TIL über den mit Siebgewebe (16) versehenen Auslassstutzen (15) des Bioreaktorgefäßes (1) von den Geweberesten abfiltriert und die Zell-Suspension mit den TIL direkt in die zweiten Stufe in ein Mäander-Bioreaktor gleicher Bauart, wie in der ersten Stufe aber mitgrößerer Besiedlungsfläche transferiert, über 12 bis 20 Tagen kultiviert, die Zellen nach der Kultivierung geerntet, gewaschen und in NaCl Lösung mit Human-Albumin resuspendiert werden und die Zell-Suspension in Infusionsbeutel abgefüllt wird.

Der erfindungsgemäße Mäander- Bioreaktor besteht aus einem rechteckigen Bioreaktorgefäß (1) aus vorzugsweise klarem Polymermaterial, das mit einem Deckel (2) steril verschlossen ist. Das Bioreaktorgefäß (1) ist in drei übereinander angeordneten Kammern (3, 4, 5) unterteilt, wobei die unterste Kammer (3) als Unterlay-Kammer, die über der Unterlay-Kammer (3) angeordnete Kammer (4) als Mäander- Perfusions-Kammer (4) und die über der Mäander- Perfusions- Kammer (4) angeordnete Kammer (5) als Overlay-Kammer ausgebildet sind. Die Unterlay-Kammer(3) und die Mäander- Perfusions- Kammer (4) sind durch eine gelochte Bodenplatte (6) mit einer darauf dicht befestigten und für Sauerstoff permeablen Folie (7) voneinander getrennt. An der Unterlay-Kammer (3) sind- Zu- und Abführungen vorhanden (13,14), die eine Durchströmung mit Gasen, insbesondere mit Gemischen aus Luft und Sauerstoff oder mit Stickstoff und Sauerstoff ermöglichen, in denen der Anteil an Sauerstoff geregelt ist. Der Sauerstoff diffundiert bevorzugt gegenüber Stickstoff durch die Gas-permeable Folie (7), die dicht auf der gelochten Bodenplatte (6) der Mäander- Perfusions- Kammer (4) befestigt ist. Bei der Durchströmung der Mänder- Perfusions- Kammer (4) gelangen während der Expansion der Zellen sowohl aus dem Overlay als auch aus dem Unterlay (durch Diffusion) geregelte Mengen an Sauerstoff in das Kulturmedium. Die Mäander-Perfusions- Kammer (4) ist mit Zu- und Abführungen für Kulturmedien (11, 12) versehen, ebenso sind in der OverlayKammer (5) Zu- und Abführung (13,14) für die Overlay- Atmosphäre sowie ein Auslassstutzen (15) vorhanden. An dem Auslassstutzen (15) ist im Innern der Overlay Kammer (5) vor dem Auslauf ein Siebgewebe (16) angeordnet. An der Abführung (12) der Mäander- Perfusions-Kammer (4) ist weiter ein Überlauf (17) für die Abführung der verbrauchten Zellbrühe vorhanden. Der Überlauf ist in seiner Höhe verstellbar.

Die Mäander- Perfusions- Kammer (4) besteht aus der Bodenplatte (6) mit auf der Oberseite (8) angeordneten streifenförmigen , die Bodenplatte (6) unterteilenden und eine mäanderförmigen Durchströmung der Mäander- Perfusions-Kammer (4) mit Gasen und Medium erzwingenden Trennwände (8),wobei der Abstand (A) der Trennwände (8) voneinander sowie von den Seiten- und Stirnwänden der Mäander-Perfusions- Kammer (4) so gewählt ist, dass sich in dem Stromfaden, in dem durch die Trennwände (8) gebildeten Gerinne, eine gemittelte laminare Oberströmung mit einer Froudezahl < 0,005 bildet. Mit zunehmender Zellzahl im Verlauf der Vermehrung der Zellmenge (TIL oder andere Zellen) wird die Zufuhr von frischem Medium kontinuierlich erhöht, wobei das erhöhte Zuführen von frischem Medium durch einen entsprechenden Algorithmus automatisch gesteuert wird. Die Bodenströmung verbleibt dabei nahe einer Froudezahl 0, wodurch eine Aufwirbelung der Zellen vermieden wird. Die beschriebenen Strömungsverhältnisse verhindern Zellstress und sorgen für eine homogene Versorgung mit Nährstoffen über die gesamte am Boden des Gerinnes aufwachsende Zellschicht. Die Overlay-Kammer (5) besitzt wie die Unterlay-Kammer Zu- und Abführungen (13, 14) für die Überströmung mit Gasen. Die OverlayKammer wird jeweils mit dem gleichen Gas/Gasgemisch wie die Unterlay-Kammer (5) durchströmt. In einem Kultivierungslauf wachsen üblicherweise 7 bis 14 Tage lang Zellen aus den Gewebestücken aus, die ausgewachsenen Zellen vermehren sich gleichzeitig. So können TIL in größerer Menge aus Tumorgewebe hergestellt werden. Aber auch TINK und weitere Zellen lassen sich so aus Geweben gewinnen. Wenn der Glukoseverbrauch im Bioreaktor pro Tag 100 bis 300 mg erreicht, werden die TIL über den mit Siebgewebe (16) versehenen Auslassstutzen (15) des Bioreaktorgefäßes (1) als Filtrat von den Geweberesten abgetrennt.

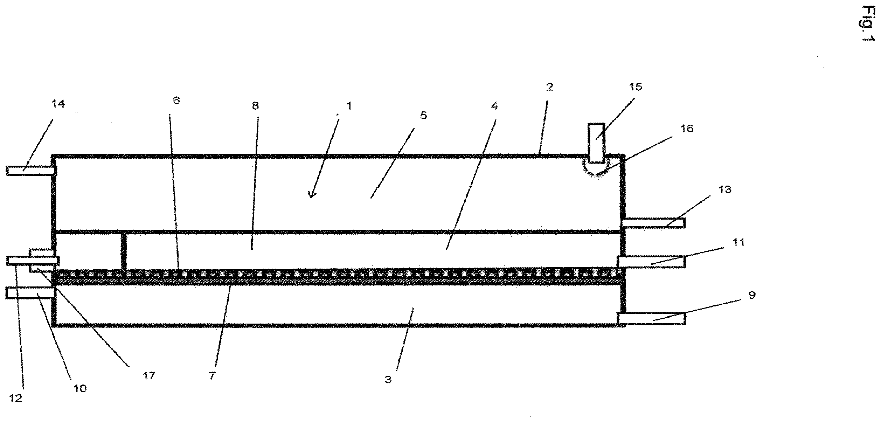

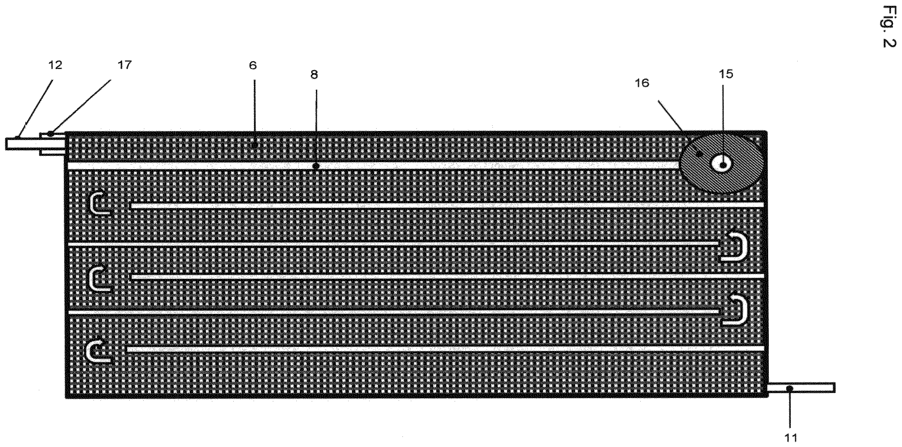

Der Mäander- Bioreaktor und das zweistufige Verfahren der zur ex- vivo Gewinnung und Vermehrung von Zellen aus Gewebeteilen werden nachfolgend näher erläutert, wobei die Fig. 1 eine schematische Schnittdarstellung des Mäander- Bioreaktors und die Fig. 2 eine schematische Draufsicht auf den Mäander- Bioreaktor zeigen, wobei die Bezugszeichen 1 bis 17 die in der nachfolgenden Liste aufgeführten Konstruktions-Teile der Bioreaktoren markieren:
- 1: Bioreaktorgefäß
- 2: Deckel
- 3: Unterlay- Kammer
- 4: Mänder- Perfusions- Kammer
- 5: Overlay- Kammer
- 6: Bodenplatte
- 7: Folie
- 8: Trennwand
- 9: Zuführung Unterlay- Atmosphäre
- 10: Abführung Unterlay- Atmosphäre
- 11: Zuführung Kulturmedium
- 12: Abführung Kulturmedium
- 13: Zuführung Overlay- Atmosphäre
- 14 1: Abführung Overlay- Atmosphäre
- 15: Auslaufstutzen
- 16: Siebgewebe
- 17: Überlauf für Zellbrühe

Ausgangsmaterial für die Herstellung von TIL oder TINK und anderen Zellen sind Gewebeanteile, die z.B. bei der operativen Entfernung solider Organtumore, ihrer Metastasen oder ihres unmittelbar umgebenden Gewebes anfallen oder für diesen Zweck entnommen werden. Eine Gewebemenge mit etwa 1 ml Volumen ist in der Regel ausreichend als Ausgangsmaterial. Die Gewebeproben werden in ein Transportgefäß mit Medium verbracht. Das Gefäß wird verschlossen bei Raumtemperatur gehalten und vom Ort der Gewebeentnahme in den Reinraumbereich für die Herstellung der Immunzell-Präparate transportiert. Die OP-Gewebestücke werden in einem Reinraum unter einer LFB in Stücke von 1 bis 2 mm3 Größe zerkleinert und in der ersten Stufe in einen Mäander- Bioreaktor transferiert. Die zerkleinerten Gewebestücke werden im Mäander-Bioreaktor gleichmäßig verteilt und im Perfusionsbetrieb kultiviert. Der Mäander Bioreaktor ist in einem GMP Breeder betriebsbereit angeschlossen und wird von der Control Unit kontinuierlich überwacht, weitgehend automatisch gesteuert und dokumentiert.

Der Mäander- Bioreaktor besteht aus einem rechteckigen Bioreaktorgefäß (1) aus vorzugsweise klarem Polymermaterial, das mit einem Deckel (2) steril verschlossen ist. Das Bioreaktorgefäß (1) ist in drei übereinander angeordneten Kammern (3, 4, 5) unterteilt, wobei die unterste Kammer (3) als Unterlay-Kammer, die über der Unterlay-Kammer (3) angeordnete Kammer (4) als Mäander-Perfusions- Kammer und die über der Mäander- Perfusions- Kammer (4) angeordnete Kammer (5) als Overlay-Kammer ausgebildet sind. Die Unterlay- Kammer(3) und die Mäander- Perfusions- Kammer (4) sind durch eine gelochte Bodenplatte (6) mit einer darauf dicht befestigten und für Sauerstoff permeablen Folie (7) voneinander getrennt. An der Unterlay-Kammer (3) sind Zu- und Abführungen vorhanden (9, 10), die eine Durchströmung mit Gasen, insbesondere mit Gemischen aus Luft und Sauerstoff oder mit Stickstoff und Sauerstoff ermöglichen, in denen der Anteil an Sauerstoff geregelt ist. Der Sauerstoff diffundiert bevorzugt gegenüber Stickstoff durch die Gas-permeable Folie (7), diedicht auf der gelochten Bodenplatte (6) der Mäander- Perfusions- Kammer (4) befestigt ist. Bei der Durchströmung der Mänder- Perfusions- Kammer (4) gelangen während der Expansion der Zellen sowohl aus dem Overlay- als auch aus dem Unterlay- Kammer (durch Diffusion) geregelte Mengen an Sauerstoff in das Kulturmedium. Die Mäander- Perfusions- Kammer (4) ist mit Zu- und Abführungen für Kulturmedien (11, 12) versehen, ebenso sind in der Overlay- Kammer (5) Zu- und Abführung (13,14) für die Overlay- Atmosphäre sowie ein Auslassstutzen (15) vorhanden. Am Auslassstutzen (15) ist im Innern der Overlay- Kammer (5) vor dem Auslauf ein Siebgewebe (16) angeordnet. An der Abführung (12) der Mäander- Perfusions-Kammer (4) ist weiter ein Überlauf (17) für die Abführung der verbrauchten Zellbrühe vorhanden. Der Überlauf ist in seiner Höhe verstellbar. Die Mänder-Perfusions- Kammer (4) besteht aus der Bodenplatte (6) mit auf der Oberseite (8) angeordneten streifenförmigen , die Bodenplatte (6) unterteilenden und eine mäanderförmigen Durchströmung der Mäander- Perfusions-Kammer (4) mit Gasen und Medium erzwingenden Trennwände (8), wobei der Abstand (A) der Trennwände (8) voneinander sowie von den Seiten- und Stirnwänden der Mäander-Perfusions- Kammer (4) so gewählt ist, dass sich in dem Stromfaden, in dem durch die Trennwände (8) gebildeten Gerinne, eine gemittelte laminare Oberströmung mit einer Froudezahl < 0,005 bildet. Mit zunehmender Zellzahl im Verlauf der Vermehrung der Zellmenge (TIL oder andere Zellen) wird die Zufuhr von frischem Medium kontinuierlich erhöht, wobei dies durch einen entsprechenden Algorithmus automatisch gesteuert wird. Die Bodenströmung verbleibt dabei nahe einer Froudezahl 0, wodurch eine Aufwirbelung der Zellen vermieden wird. Die beschriebenen Strömungsverhältnisse verhindern Zellstress und sorgen für eine homogene Versorgung mit Nährstoffen über die gesamte am Boden des Gerinnes aufwachsende Zellschicht. Die Overlay-Kammer (5) besitzt Zu- und Abführungen (13,14) für die Überströmung mit Gasen. Die Overlay-Kammer wird jeweils mit dem gleichen Gas/Gasgemisch durchströmt wie die Unterlay-Kammer (5).

In einem Kultivierungslauf wachsen üblicherweise 7 bis 14 Tage lang Zellen aus den Gewebestücken aus, die ausgewachsenen Zellen vermehren sich gleichzeitig. So können TIL in größerer Menge aus Tumorgewebe hergestellt werden. Aber auch TINK und weitere Zellen lassen sich so aus Geweben gewinnen. Wenn der Glukoseverbrauch im Bioreaktor pro Tag 100 bis 300 mg erreicht, werden die TIL über den mit Siebgewebe (16) versehenen Auslassstutzen (15) des Bioreaktorgefäßes (1) als Filtrat von den Geweberesten abgetrennt.

Ab der Verbringung der Gewebstücke in der ersten Stufe in das Bioreaktorgefäß (1) verläuft der gesamte Prozess bis zur Abfüllung aktivierter und expandierter Zellen in einem völlig geschlossenen Gefäßsystem. Kultiviert wird in geeignetem Medium, das mit einem spezifischen Gemisch aus AB Humanserum, Cytokinen, Antikörpern und irradiierten humanen Feederzellen zeitweilig supplementiert ist. Schnellere Vermehrung und Aktivierungen gelingen fallweise durch Supplemente, die auf den Oberflächen des Bioreaktorgerinnes fixiert sind. Im Fall von TIL und anderen Immunzellen werden die in dieser ersten Stufe vermehrten und separierten TIL zentrifugiert, in Einfriermedium resuspendiert und aliquotiert (rund 50 Mio. TIL pro Vial). Die Proben werden in der Gasphase über flüssigem Stickstoff gelagert.

Für klinische Anwendungen werden kryokonservierte Vials zeitlich so aufgetaut, dass in einer zweiten Expansionsstufe therapeutisch sinnvolle Mengen an TIL termingerecht produziert werden. Dazu werden nach Auswaschen vom Einfriermedium die TIL erneut in geeignetem Medium suspendiert. Die TIL-Suspension (50 bis 100 Mio. vitale Zellen) wird in einen weiteren betriebsbereit installierten Mäander- Bioreaktor transferiert. Diese zweite Prozessstufe zur weiteren Expansion von TIL wird in einem Mäander-Bioreaktor durchgeführt, der dem vorstehend beschriebenen Mäander-Bioreaktor gleicht, der aber eine größere Besiedlungsfläche besitzt. Für die weitere Vermehrung wird frisch supplementiertes Medium verwendet. Nach 12 bis 20 Tagen Expansionsdauer wachsen im Mäander-Bioreaktor regelmäßig mehr als 500 Mio. TIL hoch. Die TIL werden geerntet, gewaschen und in NaCl Lösung mit Human-Albumin resuspendiert. Die TIL-Suspension wird in einen Infusionsbeutel abgefüllt. Viablilität und Zellzahl werden am Tag der Zellernte bestimmt und weiter nach Vorgabe der Pharmakopoe, Sterilität, Markerprofil, parakrine Produktion der Zellen analysiert. Die TIL-Suspension wird bei Raumtemperatur gehalten und sofort zum klinischen Partner transportiert. Die Applikation der TIL muss spätestens nach 12 bis 24 Stunden erfolgen. Überschüssige Mengen an TIL werden kryokonserviert. Sie stehen für später notwendige werdende Herstellung von weiteren Dosen an TIL zur Verfügung, Für 500 Mio. TIL werden max.100 ml NaCl- Lösung mit Human-Albumin eingesetzt.

Mit dem erfindungsgemäßen Mäander Bioreaktor und dem erfindungsgemäßen Verfahren für die Isolierung und Vermehrung von Zellen aus Gewebeteilen von Tumoren, Metastasen und anderen Geweben es gelingt es, in einem einzigen geschlossenen Verfahrensschritt aus kleingeschnittenen Gewebestücken bestimmte Zellen sowohl in das im Bioreaktor befindliche Medium auswachsen zu lassen, als auch die Zellen gleichzeitig zu aktivieren, zu vermehren und dann von Geweberesten abzutrennen. Insbesondere können auf diese Weise tumorinfiltrierende autologe T-Lymphozyten (TIL) aus Tumorgewebe, Metastasengewebe, deren Umgebungsgewebe und aus Lymphknoten, die von Tumorzellen befallenen sind, gewonnen werden. Die gerichtete laminare Überströmung der Zellen mit Medium im Mäander- Bioreaktor führt zu schnellem Wachstum der Zellen. Im Fall von TIL werden dabei besonders die Zellen bevorzugt expandiert, die spezifische Zytotoxizität gegenüber den Tumorzellen besitzen, aus denen oder deren Umgebung sie gewonnen werden

In einem zweiten Verfahrensschritt werden in einem Bioreaktor, der dem Mäander-Bioreaktor des ersten Verfahrensschritts gleicht, der aber eine größere Besiedlungsfläche besitzt, therapeutisch sinnvolle Mengen an TIL für klinische Anwendungen produziert.

## Patentansprüche

1. Mäander- Bioreaktor bestehend aus einen rechteckigen Bioreaktorgefäß (1) aus vorzugsweise klarem Polymermaterial, das mit einem Deckel (2) steril verschlossen ist, **dadurch gekennzeichnet, dass** das Bioreaktorgefäß (1) in drei übereinander angeordneten Kammern (3,4,5) unterteilt ist und die unterste Kammer (3) als Unterlay- Kammer, die über der Unterlay-Kammer (3) angeordnete Kammer (4) als Mäander- Perfusions- Kammer und die über der Mäander- Perfusions- Kammer (4) angeordnete Kammer (5) als Overlay-Kammer ausgebildet und die Unterlay- Kammer (3) und die MäanderPerfusions- Kammer (4) durch eine gelochte Bodenplatte (6) mit einer unten an der gelochten Bodenplatte (6) angeordneten semipermeablen olie (7) voneinander getrennt sind, wobei Sauerstoff bevorzugt gegenüber anderen Gasen durch die Folie diffundiert.

2. Mäander - Bioreaktor nach Anspruch 1, **dadurch gekennzeichnet, dass** die Unterlay- Kammer (3) mit in unterschiedlicher Höhe angebrachte Zu- und Abführungen (9,10) für die Durchströmung mit Gasen, die Mäander-Perfusions- Kammer (4) mit Zu- und Abführungen für Kulturmedien (11, 12) und die Overlay- Kammer (5) mit Zu- und Abführungen (13,14) für Overlay-Gase und mit einem Auslassstutzen (15), an dem im Innern der Overlaykammer (5) vor dem Auslauf ein Siebgewebe (16) angeordnet ist, trägt, wobei an der Zuführung (11) der Mäander- Bioreaktor- Kammer (4) ein angeordnete Überlauf (17) für die verbrauchte Zellbrühe in seiner Höhe verstellbar angeordnet ist.

3. Mäander- Bioreaktor nach Anspruch 1-2 **dadurch gekennzeichnet, dass** die Mänder- Perfusions- Kammer (4) aus der Bodenplatte (6) mit auf der Oberseite (8) angeordneten streifenförmigen , die Bodenplatte (6) unterteilenden und eine mäanderförmige Durchströmung der Mäander-PerfusionsKammer (4) mit Medium erzwingenden Trennwänden (8) besteht, wobei der Abstand (A) der Trennwände (8) voneinander sowie von den Seiten- und Stirnwänden der Mäander- Perfusions- Kammer (4) so gewählt ist, dass in dem Stromfaden, in den durch die Trennwände (8) gebildeten Gerinne, eine gemittelte laminare Oberströmung mit einer Froudezahl nicht größer als 0,005 und eine Bodenströmung mit einer Froudezahl nahe 0 ausgebildet ist, die bei wachsender Zellmenge sowohl eine homogene Konzentration an Nährstoffen, als auch keine Aufwirbelung verursachende Strömung im überströmenden Medium der sedimentierten Zellen sicher stellt.

4. Mäander- Bioreaktor nach Anspruch 1-3 **dadurch gekennzeichnet, dass** über den Überlauf (17) für die Zellbrühe (mit nicht näher dargestellten Absperrvorrichtungen) mehrere Mäander- Bioreaktoren modulartig miteinander verbunden werden können.

5. Verfahren die ex- vivo Gewinnung von Zellen im Mäander- Bioreaktor gemäß Anspruch 2 , wobei die Zellen in einer ersten Stufe des Verfahrens aus zerkleinerten Gewebeteilen in das im Bioreaktor befindliche Medium auswachsen, gleichzeitig aktiviert und vermehrt und nach Erreichen einer bestimmten Dichte an ausgewachsenen und expandierten Zellen von den Gewebeteilen separiert werden, **dadurch gekennzeichnet, dass** die Gewebeteile, die von Tumorzellen befallenen sind, aus Anlass von Organtumor- oder Metastasen-Operationen oder Entfernung von deren unmittelbar umgebenden Geweben oder auch aus Lymphknoten entnommen und in der ersten Stufe des Verfahrens in ein geschlossenes Bioreaktorgefäß (1) verbracht werden, wobei im Bioreaktorgefäß (1) die Medium gerichtet perfundiert werden, das Medium mit einem Gemisch aus AB Humanserum, Cytokinen, Antikörpern und irradiierten humanen Feederzellen zeitweilig supplementiert wird und nach 7 bis 14 Tagen Expansionsdauer die ausgewachsenen und vermehrten TIL über den mit Siebgewebe (16) versehenen Auslassstutzen (15) des Bioreaktorgefäßes (1) von den Geweberesten abfiltriert und in reiner Form geerntet, die so separierten TIL zentrifugiert und in Einfriermedium in Vials resuspendiert, aliquotiert und kryokonserviert werden.

6. Verfahren nach Anspruch 5, **dadurch gekennzeichnet, dass** tumorinfiltrirende Lymphozyten (TIL) oder tumorinfiltrierende NK-Zellen (TINK) und weitere Zellarten im Mäander Bioreaktor nach Anspruch 1 in entsprechend supplementierten Medien kultiviert werden.

7. Verfahren nach Anspruch 5 **dadurch gekennzeichnet, dass** durch Zusatz geeigneter Supplemente (Antikörper) die Vermehrung von TIL gezielt verhindert wird und auf diese Weise TINK aus den zerkleinerten Gewebeteilen vermehrt und in reiner Form geerntet werden, wobei die über das Siebgewebe (16) TINK abfiltriert und in reiner Form geerntet, die so separierten TINK zentrifugiert und in Einfriermedium in Vials resuspendiert, aliquotiert und kryokonserviert werden.

8. Verfahren nach Anspruch 6 und 7, **dadurch gekennzeichnet, dass** die kryokonservierten Vials für die weitere Expansion aufgetaut werden und das Einfriermedium ausgewaschen wird, wobei die Zellen in ein entsprechendes, frisch supplementiertes Medium resuspendiert werden und die ZellSuspension in einer zweiten Stufe in ein Mäander- Bioreaktor gleicher Bauart, wie in der ersten Stufe aber mit größerer Besiedlungsfläche transferiert, über 12 bis 20 Tagen kultiviert werden, die Zellen nach der Kultivierung geerntet, gewaschen und in NaCl Lösung mit Human-Albumin resuspendiert werden und die Zell-Suspension in Infusionsbeutel abgefüllt wird.

9. Verfahren nach Anspruch 1, **dadurch gekennzeichnet, dass** das Verhältnis der Besiedelungsfläche in diesem größeren Mäander-Bioreaktor der zweiten Stufe gegenüber dem in Anspruch 1 beschriebenen kleineren Mäander-Perfusions-Bioreaktor 3 zu 1 bis 20 zu 1 betragen kann.

10. Verfahren nach Anspruch 6-9, **dadurch gekennzeichnet, dass** vermehrte TIL über den mit Siebgewebe (16) versehenen Auslassstutzen (15) des Bioreaktorgefäßes von den Geweberesten abfiltriert und die Zell-Suspension mit den TIL direkt in die zweiten Stufe in ein Mäander- Bioreaktor gleicher Bauart, wie in der ersten Stufe, aber mitgrößerer Besiedlungsfläche transferiert, über 12 bis 20 Tagen kultiviert, die Zellen nach der Kultivierung geerntet, gewaschen und in NaCl Lösung mit Human-Albumin resuspendiert werden und die Zell-Suspension in Infusionsbeutel abgefüllt.

## Claims

1. Meander bioreactor consisting of a rectangular bioreactor vessel (1) made of preferably clear polymermaterial, which is sterile closed with a lid (2), **characterized in that** the bioreactor vessel (1) is divided into three chambers (3, 4, 5) arranged one above the other and the lowest chamber (3) as an underlay chamber, the chamber (4) arranged above the underlay chamber (3) as a meander perfusion chamber and the chamber (5) arranged above the meander perfusion chamber (4) as Overlay chamber is formed and the underlay chamber (3) and the meander perfusion chamber (4) are separated from one another by a perforated base plate (6) with a semi-permeable film (7) arranged at the bottom of the perforated base plate (6), wherein Oxygen diffuses through the film preferentially over other gases.

2. Meander bioreactor according to claim 1, **characterized in that** the underlay chamber (3) with inlets and outlets (9, 10) attached at different heights for the flow of gases, the meander perfusion chamber (4) with inlet and discharges for culture media (11, 12) and the overlay chamber (5) with inlets and outlets (13,14) for overlay gases and with an outlet nozzle (15) on which inside the overlay chamber (5) before The outlet carries a sieve fabric (16), an overflow (17) for the used cell broth being adjustable in height at the inlet (11) of the meander bioreactor chamber (4).

3. Meander bioreactor according to claims 1-2, **characterized in that** the meander perfusion chamber (4) consists of the base plate (6) with strip-shaped, arranged on the top (8), dividing the base plate (6) and a meander-shaped flow through the Perfusion chamber (4) with medium-enforcing partitions (8), the distance (A) of the partitions (8) from each other and from the side and end walls of the meander perfusion chamber (4) is chosen so that in the Stream filament, in the channel formed by the dividing walls (8), an averaged laminar upper flow with a Froude number not greater than 0.005 and a bottom flow with a Froude number close to 0 is formed, which with increasing cell count both a homogeneous concentration of nutrients and no turbulence Causing flow in the medium flowing over the sedimented cells ensures.

4. Meander bioreactor according to claims 1-3, **characterized in that** several meander bioreactors can be connected to one another in modular fashion via the overflow (17) for the cell broth (with shut-off devices not shown in detail).

5. Method for the ex vivo extraction of cells in the meander bioreactor according to claim 1, wherein the cells in a first stage of the method outgrow from comminuted tissue into the medium located in the bioreactor, simultaneously activated and increased and after reaching a certain density of fully grown and expanded Cells are separated from the tissue parts, **characterized in that** the tissue parts that are affected by tumor cells are removed on the occasion of organ tumor or metastasis operations or removal of their immediately surrounding tissues or from lymph nodes and in the first stage of the procedure in a closed bioreactor vessel (1), the medium being perfused in a directed manner in the bioreactor vessel (1), the medium being temporarily supplemented with a mixture of AB human serum, cytokines, antibodies and irradiated human feeder cells and after 7 to 14 days of expansion the fully grown and multiplied TIL ü The tissue residues are filtered off via the outlet connection (15) of the bioreactor vessel (1) provided with sieve fabric (16) and harvested in pure form, the TIL thus separated are centrifuged and resuspended in freezing medium in vials, aliquoted and cryopreserved.

6. Process according to Claim 5, **characterized in that** tumor-infiltrating lymphocytes (TIL) or tumor-infiltrating NK cells (TINK) and other cell types are cultivated in the meandering bioreactor according to Claim 1 in appropriately supplemented media.

7. Method according to claim 5, **characterized in that** by adding suitable supplements (antibodies) the reproduction of TIL is specifically prevented and in this way TINK is multiplied from the comminuted tissue parts and harvested in pure form, the TINK being filtered off via the sieve fabric (16) and harvested in pure form, the so separated TINK centrifuged and resuspended in freezing medium in vials, aliquoted and cryopreserved.

8. Method according to claims 6 and 7, **characterized in that** the cryopreserved vials are thawed for further expansion and the freezing medium is washed out, the cells being resuspended in a corresponding, freshly supplemented medium and the cell suspension in a second stage in a meander - Bioreactor of the same design, as transferred in the first stage but with a larger settlement area, can be cultivated for 12 to 20 days, the cells are harvested after cultivation, washed and resuspended in NaCl solution with human albumin and the cell suspension is filled into infusion bags becomes.

9. Method according to claim 1, **characterized in that** the ratio of the colonization area in this larger meander bioreactor of the second stage compared to the smaller meander perfusion bioreactor described in claim 1 can be 3: 1 to 20: 1.

10. Method according to claims 6-9, **characterized in that** increased TIL is filtered off from the tissue residues via the outlet connection (15) of the bioreactor vessel provided with sieve fabric (16) and the cell suspension with the TIL is transferred directly to the second stage in a meander bioreactor Design as in the first stage, but transferred with a larger colonization area, cultivated for 12 to 20 days, the cells harvested after cultivation, washed and resuspended in NaCl solution with human albumin and the cell suspension is filled into infusion bags.

## Revendications

1. Bioréacteur à méandre constitué d'un récipient de bioréacteur rectangulaire (1) en matériau polymère de préférence transparent, qui est stérile fermé avec un couvercle (2), **caractérisé en ce que** le récipient de bioréacteur (1) est divisé en trois chambres (3, 4, 5) disposées l'une au-dessus de l'autre et la chambre la plus basse (3) en tant que chambre de sous-couche, la chambre (4) disposée au-dessus de la chambre de sous-couche (3) en tant que chambre de perfusion en méandre et la chambre (5) disposée au-dessus de la chambre de perfusion en méandre (4) en tant que Une chambre de recouvrement est formée et la chambre de sous-couche (3) et la chambre de perfusion en méandre (4) sont séparées l'une de l'autre par une plaque de base perforée (6) avec un film semi-perméable (7) disposé au fond de la plaque de base perforée (6), dans laquelle L'oxygène diffuse à travers le film de préférence sur les autres gaz.

2. Bioréacteur à méandre selon la revendication 1, **caractérisé en ce que** la chambre de sous-couche (3) avec entrées et sorties (9, 10) fixées à différentes hauteurs pour l'écoulement des gaz, la chambre de perfusion à méandre (4) avec entrée et des décharges pour les milieux de culture (11, 12) et la chambre de recouvrement (5) avec des entrées et des sorties (13, 14) pour les gaz de recouvrement et avec une buse de sortie (15) sur laquelle l'intérieur de la chambre de recouvrement (5) avant La sortie porte un tamis (16), un trop-plein (17) pour le bouillon de cellules utilisé étant réglable en hauteur à l'entrée (11) de la chambre de bioréacteur en méandre (4).

3. Bioréacteur à méandre selon les revendications 1 et 2, **caractérisé en ce que** la chambre de perfusion à méandre (4) est constituée de la plaque de base (6) en forme de bande, disposée sur la face supérieure (8), divisant la plaque de base (6) et d'un écoulement en forme de méandre à travers le méandre Chambre de perfusion (4) avec cloisons (8) renforçant le milieu, la distance (A) des cloisons (8) les unes des autres et des parois latérales et d'extrémité de la chambre de perfusion en méandre (4) est choisie de telle sorte que dans le Filament de flux, dans le canal formé par les parois de séparation (8), un écoulement supérieur laminaire moyen avec un nombre de Froude non supérieur à 0,005 et un écoulement de fond avec un nombre de Froude proche de 0 se forme, qui avec l'augmentation du nombre de cellules à la fois une concentration homogène de nutriments et aucune turbulence Causer un écoulement dans le milieu circulant sur les cellules sédimentées assure.

4. Bioréacteur à méandre selon les revendications 1 à 3, **caractérisé en ce que** plusieurs bioréacteurs à méandre peuvent être connectés les uns aux autres de manière modulaire via le trop-plein (17) pour le bouillon cellulaire (avec des dispositifs de fermeture non représentés en détail).

5. Procédé pour l'extraction ex vivo de cellules dans le bioréacteur à méandres selon la revendication 1, dans lequel les cellules dans une première étape du procédé dépassent des parties de tissu broyées dans le milieu dans le bioréacteur, simultanément activées et augmentées et après avoir atteint une certaine densité de croissance complète et expansée. Les cellules sont séparées des parties de tissu, **caractérisé en ce que** les parties de tissu qui sont affectées par les cellules tumorales sont retirées à l'occasion d'opérations de tumeur d'organe ou de métastase ou de prélèvement de leurs tissus immédiatement environnants ou également de ganglions lymphatiques et dans la première étape de la procédure dans un récipient de bioréacteur fermé (1), le milieu étant perfusé de manière dirigée dans le récipient de bioréacteur (1), le milieu étant temporairement complété par un mélange de sérum humain AB, de cytokines, d'anticorps et de cellules nourricières humaines irradiées et après 7 à 14 jours d'expansion, le milieu est complètement développé et multiplié TIL ü Les résidus de tissu sont filtrés via la connexion de sortie (15) du récipient de bioréacteur (1) muni de tissu de tamis (16) et récoltés sous forme pure, les TIL ainsi séparés sont centrifugés et remis en suspension dans du milieu de congélation dans des flacons, aliquotés et cryoconservés

6. Procédé selon la revendication 5, **caractérisé en ce que** des lymphocytes infiltrant les tumeurs (TIL) ou des cellules NK infiltrant les tumeurs (TINK) et d'autres types cellulaires sont cultivés dans le bioréacteur sinueux selon la revendication 1 dans des milieux convenablement supplémentés.

7. Procédé selon la revendication 5, **caractérisé en ce que**, en ajoutant des suppléments appropriés (anticorps), la reproduction de TIL est spécifiquement empêchée et de cette manière TINK est multiplié à partir des parties de tissu broyé et récolté sous forme pure, le TINK étant filtré via le tissu de tamis (16) et récolté sous forme pure, le TINK ainsi séparé centrifugé et remis en suspension dans un milieu de congélation dans des flacons, aliquoté et cryoconservé.

8. Procédé selon les revendications 6 et 7, **caractérisé en ce que** les flacons cryoconservés sont décongelés pour une expansion supplémentaire et le milieu de congélation est lavé, les cellules étant remises en suspension dans un milieu correspondant fraîchement supplémenté et la suspension cellulaire dans une deuxième étape dans un méandre. - Le bioréacteur de même conception, tel que transféré dans la première étape mais avec une zone de peuplement plus grande, peut être cultivé pendant 12 à 20 jours, les cellules sont récoltées après culture, lavées et remises en suspension dans une solution de NaCI avec de l'albumine humaine et la suspension cellulaire est versée dans des sacs de perfusion devient.

9. Procédé selon la revendication 1, **caractérisé en ce que** le rapport de la zone de colonisation dans ce bioréacteur à méandre plus grand du deuxième étage par rapport au bioréacteur à perfusion méandre plus petit décrit dans la revendication 1 peut être de 3: 1 à 20: 1.

10. Procédé selon les revendications 6 à 9, **caractérisé en ce que** le TIL augmenté est filtré des résidus de tissu via la connexion de sortie (15) du récipient du bioréacteur muni d'un tissu de tamis (16) et la suspension cellulaire avec le TIL est transférée directement au deuxième étage dans un bioréacteur à méandres. Conception comme dans la première étape, mais transférées avec une plus grande zone de colonisation, cultivées pendant 12 à 20 jours, les cellules récoltées après culture, lavées et remises en suspension dans une solution de NaCI avec de l'albumine humaine et la suspension cellulaire est remplie dans des sacs de perfusion.
